(19) **Europäisches Patentamt**

**European Patent Office**

**Office européen des brevets**

(11) Publication number: **0 115 065**
**B1**

(12) **EUROPEAN PATENT SPECIFICATION**

(45) Date of publication of patent specification: **06.09.89**

(51) Int. Cl.⁴: $H\ 01\ B\ 3/20$

(21) Application number: **83113081.0**

(22) Date of filing: **23.12.83**

(60) Divisional application 88104568 filed on 22.03.88.

(54) Process for preparing oil-containing electrical appliances.

(30) Priority: **25.12.82 JP 233238/82**
**20.07.83 JP 132331/83**

(43) Date of publication of application:
**08.08.84 Bulletin 84/32**

(45) Publication of the grant of the patent:
**06.09.89 Bulletin 89/36**

(84) Designated Contracting States:
**CH DE FR GB IT LI NL**

(56) References cited:
**EP-A-0 103 868**
**FR-A-2 485 563**
**FR-A-2 514 935**

(73) Proprietor: **NIPPON PETROCHEMICALS COMPANY, LIMITED**
**3-1, Uchisaiwaicho 1-chome Chiyoda-ku Tokyo (JP)**

(72) Inventor: **Sato, Atsushi**
**15-23 Yakumo 2-chome Meguro-ku Tokyo (JP)**
Inventor: **Endo, Keiji**
**4-2 Ohkubo 3-chome Konan-ku Yokohama-shi Kanagawa-ken (JP)**
Inventor: **Kawakami, Shigenobu**
**15-10 Yawata 1-chome Ichikawa-shi Chiba-ken (JP)**
Inventor: **Yanagishita, Hitoshi**
**236 Mineokacho 2-chome Hodogaya-ku Yokohama-shi Kanagawa-ken (JP)**
Inventor: **Hayashi, Shozo**
**4-3 Ohkubo 3-chome Konan-ku Yokohama-shi Kanagawa-ken (JP)**

(74) Representative: **Schübel-Hopf, Ursula et al**
**Strehl Schübel-Hopf Groening Schulz Patentanwälte Maximilianstrasse 54 Postfach 22 14 55 D-8000 München 22 (DE)**

Courier Press, Leamington Spa, England.

# EP 0 115 065 B1

**Description**

This invention relates to a process for preparing oil-containing electrical appliances.

The electrical insulating substances obtained by the process of the invention are quite suitable for use in oil-filled electrical appliances in which insulating materials or dielectric materials made of plastics such as polyolefin are at least partly employed.

Electrical appliances such as oil-filled capacitors, oil-filled power cables and transformers have recently been made to withstand high electric voltages while being small in size. With this tendency, various kinds of plastics films are used together with conventional insulating paper.

There are several electrical insulating oils known which are filled or used for impregnating these electrical appliances. However, the conventional electrical insulating oils such as refined mineral oils, polybutenes, alkylbenzenes and polychlorinated biphenyls have several drawbacks. For example, the use of polychlorinated biphenyls was discontinued because it constitutes a public health hazard. Furthermore, the conventional electrical insulating oils are not satisfactorily compatible with the above-mentioned plastics materials, such as polyolefin films, which are recently being used in oil-filled electrical appliances.

With the requirements of high-voltage durability and size reduction, it is necessary that the electrical insulating oil has a high dielectric breakdown voltage, a low dielectric loss tangent, and good hydrogen gas absorbing capacity.

The hydrogen gas absorbing capacity indicates the stability of the insulating oil against corona discharge (partial discharge) under high electric voltage conditions. The higher the gas-absorbing capacity, the smaller the likelihood of corona discharge, which leads to the advantage of the insulating oil having excellent stability or durability.

Meanwhile, in order to meet the requirement of high-voltage use, films of plastics material such as polyolefin films, polystyrene films and polyester films are used to either partially or completely replace the conventional insulating paper as insulating materials or dielectric materials for electrical appliances such as oil-filled electric cables and capacitors. In view of their dielectric strength, dielectric loss tangent and dielectric constant, polyolefin films, especially polypropylene and cross-linked polyethylene films, are preferred as those insulating or dielectric materials.

When these polyolefin films are impregnated with insulating oils, some oils cause the films to swell to some extent. If a film becomes swollen, the thickness of the insulating layer increases. As a result, the resistance to the flow of insulating oil increases in electrical cables, and insufficient impregnation with insulating oil occurs in electric capacitors, causing the formation of voids (unimpregnated portions) and undesirably lowers the corona discharge voltage.

In connection with the above-mentioned conventional electrical insulating oils, the values of the dielectric breakdown voltages (BDV) and the dielectric loss tangents (tan δ) are satisfactory to a certain extent, but the hydrogen gas absorbing capacity or corona discharge characteristics and the stability of the dimensions of polypropylene films are not satisfactory.

In view of the above-described state of the art, it is the primary object of this invention to provide a process for preparing oil-containing electrical appliances that have excellent corona discharge characteristics, dielectric breakdown voltage and other advantageous electrical characteristics, and to maintain a long service life.

EP—A—0 103 868, a prior art document under Article 54 (3), relates to an electrical insulating oil comprising diaryl alkanes in a mixture with mono- and/or diolefins having two condensed or non-condensed aromatic nuclei, i.e. a mixture of two distinct compounds, which are separately prepared in advance and mixed in order to produce the insulating oil.

By the present invention it has been found that the electrical characteristics of a by-product oil consisting of a mixture of several hydrocarbons may be improved by a simple dehydrogenation treatment. Such process results in a dehydrogenation product which constitutes a mixture of several different compounds, which is not comparable with the above-stated prior art insulating oil, which constitutes a mixture of two distinct components.

The present invention relates to a process for preparing an electrical appliance which contains or is impregnated with an electrical insulating oil wherein the apparatus is filled or impregnated with a dehydrogenated reaction mixture containing more than 0.5% by weight of an aromatic monoolefin or diolefin having two condensed or noncondensed aromatic nuclei obtained by dehydrogenating an aromatic hydrocarbon or hydrocarbons each having two condensed or noncondensed aromatic nuclei and aliphatic or alicyclic hydrocarbon residual group or groups having two or more carbon atoms at a temperature in the range of 350 to 650°C in the presence of a dehydrogenation catalyst, without further adding a diarylalkane.

In the present invention, an aromatic hydrocarbon having two condensed or noncondensed aromatic nuclei is used as a raw material. The above aromatic hydrocarbon can include fractions obtained by a distillation or separation step. The aromatic hydrocarbon can be any compound in which at least one olefinic double bond is produced by dehydrogenation. In other words, the aromatic hydrocarbon is a compound that has two condensed or noncondensed aromatic nuclei and at least one aliphatic or alicyclic hydrocarbon residual group having two or more carbon atoms. These hydrocarbon residual groups are mono-valent or poly-valent hydrocarbon groups that are derived from alkanes such as ethane, propane,

2

butane, pentane, hexane, heptane and octane, and cycloalkanes such as cyclopentane and cyclohexane. However, it should be noted that the aromatic hydrocarbon having a molecular weight of about 500 or less is generally preferable because the dehydrogenation is easy and the electrical properties of the obtained product are good.

The aromatic hydrocarbons are exemplified by diarylalkane, diarylcycloalkane, arylindane, alkylbiphenyl, cycloalkylbiphenyl, alkylnaphthalene and cycloalkylnaphthalene.

The diarylalkane and diarylcycloalkane are exemplified by 1,1 - diarylalkanes such as 1,1 - diphenylethane, 1 - phenyl - 1 - tolylethane, 1 - phenyl - 1 - xylylethane and 1 - phenyl - 1 - ethylphenylethane; 1,2 - diarylalkanes such as 1 - phenyl - 2 - ethylphenylethane and 1 - phenyl - 2 - isopropylphenylethane; and diphenylcyclohexane.

The alkylbiphenyl and cycloalkylbiphenyl are exemplified by monoisopropylbiphenyl, diisopropylbiphenyl and cyclohexylbiphenyl.

The alkylnaphthalene and cycloalkylnaphthalene are exemplified by ethylnaphthalene, isopropylnaphthalene and diisopropylnaphthalene.

As a raw material for preparing the electrical appliance according to the process of this invention, any fraction containing the above defined diaromatic hydrocarbon can be used. For example, the fraction containing the diaromatic hydrocarbons that is derived from a process to prepare directly the above diaromatic hydrocarbon can be employed.

Besides the above fraction, there is a fraction that is obtained in the alkylation process to prepare alkylated aromatic hydrocarbons. More particularly, the fraction is obtained by reacting aromatic hydrocarbons with an alkylating agent in the presence of an alkylation catalyst to obtain an alkylation product containing mono- and polyalkylated aromatic hydrocarbons, diarylalkanes, heavier products, and unreacted materials, and then separating mono- and polyalkylated monoaromatic hydrocarbons, aromatic hydrocarbons having two condensed or noncondensed aromatic nuclei and an aliphatic or alicyclic hydrocarbon residual group or groups having two or more carbon atoms and unreacted materials from the above-obtained alkylation product.

The above alkylation process to prepare various aromatic hydrocarbons is explicitly described in the European Patent Application No. 88104568.6.

Through the above-mentioned reaction, an alkylation product containing unreacted materials such as benzene and ethylene, mono- and polyethylbenzene, 1,1 - diphenylethane, 1 - phenyl - 1 - ethylphenylethane, 1,1 - di(ethylphenyl)ethane and heavier products can be obtained.

This alkylation product is then subjected to usual steps of settling and filtration to remove any alkylation catalyst. After that the filtrate is rinsed with water and neutralized.

In the next step, the unreacted benzene and monoaromatic hydrocarbons such as ethylbenzene and polyethylbenzene are removed from the alkylation product through a conventional method such as distillation. The thus obtained residual fraction contains an asphaltic substance that is so far used only as fuel or the like. This asphaltic substance is then removed by a separation process such as distillation, as a distillation bottom to obtain a fraction that is to be subjected to dehydrogenation in accordance with this invention.

In the case of alkylation of benzene or toluene with ethylene, the above fraction has boiling points in the range of about 255 to 420°C, preferably about 260 to 400°C, and more preferably about 268 to 400°C. The substances contained in the fraction of this boiling range are 1,1 - diphenylethane, 1 - phenyl - 1 - ethylphenylethane, 1,1 - di(ethylphenyl)ethane, and heavier products.

The refining treatment, such as neutralization, to remove the residue of the alkylation catalyst can be carried out before or after the dehydrogenation. This refining treatment is done by using an appropriate organic or inorganic basic substance. As the basic substances, there are alkali metals of group I and alkaline earth metals of group II of the periodic table, their oxides and their hydroxides. More particularly, lithium, sodium, potassium, magnesium, calcium, strontium and barium, their oxides and their hydroxides are preferable. The refining treatment can be performed in ordinary manner, and also it is possible to use the above metallic oxide in the distillation step. Further, the refining treatment can be carried out by using absorbents such as clay, silica, alumina and silica · alumina.

The above-described starting materials are dehydrogenated, and the obtained dehydrogenation product or dehydrogenated reaction mixture is employed in this invention.

The dehydrogenation is carried out in the presence of a catalyst, in which any known dehydrogenation catalyst can be used. For instance, exemplified as the catalysts are oxides of metals such as chromium, iron, copper, potassium, magnesium and calcium, precious metals such as platinum and palladium, or those metal oxides or precious metals that are supported on a carrier of alumina or the like.

The reaction temperature of dehydrogenation is in the range of 350 to 650°C, preferably 400 to 600°C. When the dehydrogenation is carried out through a continuous fixed bed process, the LHSV (liquid hourly space velocity) is in the range of 0.2 to 10, preferably 0.5 to 3.0.

In the dehydrogenation, an inert gas such as steam can be introduced into the reaction system. Further, if necessary, a suitable diluent can be used. However, when the rate of dehydrogenation is not so high, raw materials themselves conveniently serve as a diluent.

In the dehydrogenation process of this invention, the above catalysts and reaction conditions are properly selected in order to suppress the occurrence of side effects such as cracking (decomposition) or

3

polymerization. However, it is necessary that the dehydrogenation must be so carried out as to produce more than 0.5 wt.%, preferably more than 5.0 wt.% in the dehydrogenated reaction mixture, of the aromamic monoolefin or diolefin having two condensed or noncondensed aromatic nuclei. When the content of the above aromatic olefin is less than 0.5 wt.%, this invention cannot be expected to be effective.

Incidentally, complete dehydrogenation is generally difficult and it is accompanied by decomposition and polymerization at higher conversion rates. Heavier components produced by polymerization and lighter aliphatic or cycloaliphatic components produced by decomposition are not of use, however, the aromatics having two aromatic nuclei sometimes produced by partial decomposition, are rather desirable components in the present invention, because the synergistic effect among produced olefins and unreacted starting material and/or the above partially decomposed aromatics can be expected.

Therefore, after the reaction, hydrogen, and if necessary, lighter components such as aliphatic or cycloaliphatic hydrocarbons produced by side reaction such as decomposition and heavier components produced by polymerization are removed by distillation, thereby obtaining the dehydrogenated reaction mixture, that is, the electrical insulating substance of this invention.

From the above-described electrical insulating substance, an electrical insulating oil can be prepared by the following procedure:

If the obtained reaction mixture has a proper viscosity and the content of the above-mentioned aromatic olefins in the mixture is appropriate, the dehydrogenated reaction mixture can be used as it stands as an electrical insulating oil. When the viscosity is too high or the content of the aromatic olefins is too large, the dehydrogenated reaction mixture is mixed and dissolved together with a proper quantity of conventional electrical insulating oil or oils to prepare easily an excellent electrical insulating oil. Furthermore, when the above electrical insulating substance is a solid material at ordinary temperatures but melts at elevated temperatures of usual impregnation, the electrical insulating substance can be used by heating and fusing it for impregnation. In another manner of use, the electrical insulating substance is dissolved into any suitable electrical insulating oil having dissolving power, thereby obtaining an excellent electrical insulating substance in accordance with this invention.

Incidentally, when the above-mentioned aromatic olefins prepared by dehydrogenation are used, they can be isolated through a separating process such as distillation, extraction or recrystallization. The boiling points of components are generally close to each other and any undesirable effect such as polymerization of the aromatic olefins is hardly observed. In addition, the electrical appliances made by using the dehydrogenation product show quite excellent electrical performance because of a synergistic effect. Accordingly, the electrical insulating oil is preferably prepared by using the electrical insulating substance without applying the above isolation process of the aromatic olefins. Therefore, it is preferable to use the dehydrogenated reaction mixture alone or the combination of the mixture with other electrical insulating oils.

As long as the electrical insulating substance prepared according to the invention can be dissolved, any quantity of the following conventional electrical insulating oils can be mixed together. Such insulating oils are exemplified by mineral oils, olefin oligomers such as polybutene alkyl- or cycloalkylbenzenes such as dodecylbenzene and dicyclohexylbenzene, animal and vegetable oils such as castor oil as a triglyceride, phthalic esters such as dioctylphthalate, and silicone oil.

In addition to the above electrical insulating oils, saturated compounds having two condensed or noncondensed aromatic nuclei and aromatic olefins having at least two condensed or noncondensed aromatic nuclei can be used by mixing them with the electrical insulating substance.

The saturated compounds having two condensed or noncondensed aromatic nuclei are exemplified by diphenylmethane; lower alkyl derivatives of diphenylmethane such as benzyltoluene; 1,1 - diphenylethane, 1,2 - diphenylethane or their lower alkyl derivatives such as 1 - phenyl - 1 - xylylethane, 1 - phenyl - 1 - ethylphenylethane, 1 - phenyl - 1 - tolylethane, 1 - phenyl - 1 - isopropylphenylethane, 1 - phenyl - 1 - trimethylphenylethane, 1,1 - di(ethylphenyl)ethane, 1 - phenyl - 2 - ethylphenylethane, 1 - phenyl - 2 - isopropylphenylethane, and 1,2 - dixylylethane; diarylalkanes and diarylcycloalkanes such as 1,3 - diphenylbutane, 2,4 - diphenyl - 2 - methylpentane and diphenylcyclohexane; alkylarylindane such as methylphenylindane; biphenyl; alkyl- or cycloalkylbiphenyls such as mono- or diisopropylbiphenyl and cyclohexylbiphenyl; alkylnaphthalenes such as mono- or diisopropylnaphthalene; ethers such as ditolyl ether, dixylyl ether, dibenzyl ether, bis (α - methylbenzyl)ether and diaryl thioether; and triarylalkanes such as dibenzyltoluene, distyrenated xylene, bisphenetyltoluene, terphenyl, styrenated naphthalene, and triphenylhexane.

The above-mentioned aromatic olefins having at least two condensed or noncondensed aromatic nuclei are exemplified by 1,1 - diphenylethylene; stilbene; unsaturated dimers or trimers of styrenes including styrene, vinyltoluene, α - methylstyrene and isopropenyltoluene, such as 1,3 - diphenylbutene - 1, and 2,4 - diphenyl - 4 - methylpentene - 1; vinylphenyl - phenylalkanes and their lower alkyl derivatives such as 1 - vinylphenyl - 1 - phenylethane, 1 - isopropenylphenyl - 1 - phenylethane and vinylphenyl - phenylmethane; and other aromatic monoolefins such as isopropenylbiphenyl and isopropenylnaphthalene.

One or a mixture of two or more of the above known insulating oils can be used in combination with the dehydrogenation product prepared according to this invention.

In order to improve further the oxidation stability, several known antioxidants can be added to the

4

electrical insulating oil. As such antioxidants, there are phenol compounds like 2,6 - di - tert - butyl - p - cresol, 2,2' - methylenebis(4 - methyl - 6 - tert - butylphenol), 4,4' - butylidenebis(3 - methyl - 6 - tert - butylphenol), 4,4' - thiobis(3 - methyl - 6 - tert - butylphenyl), stearyl - β - (3,5 - di - tert - butyl - 4 - hydroxyphenol)propionate, tetrakis[methylene - 3(3',5' - di - tert - butyl - 4' - hydroxyphenyl) - propionate]methane, 1,3,5 -trimethyl - 2,4,6 -tris(3,5 - di - tert - butyl - 4 - hydroxybenzyl)benzene, and 1,1,3 - tris(2 - methyl - 4 - hydroxy - 5 - tert - butylphenol)butane; sulfur compounds such as dilauryl thiodipropionate, distearyl thiodipropionate, laurylstearyl thiodipropionate, and dimyristyl thiodipropionate; and phosphorous compounds such as triisodecylphosphite, diphenylisodecylphosphite, triphenylphosphite, and trinonylphenylphosphite.

These antioxidants can be added to the electrical insulating substance singly or in combination of two kinds or more. The additional quantity of the antioxidant is 0.001 to 5% by weight and preferably 0.01 to 2.0% by weight of the electrical insulating substance.

Furthermore, in order to impart a nonflammable property and other desirable effects to the electrical insulating substance of this invention, several known additives such as phosphoric esters and epoxy compounds can be added to the electrical insulating substance.

When the electrical insulating substance of this invention is used as an electrical insulating oil, it shows excellent compatibility with insulating materials made of plastics such as polyolefins. Accordingly, when the electrical insulating substance is used for impregnating oil-filled electrical appliances having dielectric or insulating materials partially or totally made of plastics, high-tension, light-weight and long-life electrical appliances can be provided. Furthermore, the electrical insulating substance prepared according to this invention can be mixed with or dissolved in known electrical insulating oils including hydrocarbons having at least two condensed or noncondensed aromatic nuclei such as diarylalkanes, alkylbiphenyls and alkylnaphthalenes, and as a result, similar advantages can be attained with the synergistic effect of combined components.

Accordingly, the electrical insulating substance prepared according to this invention, or the electrical insulating oil containing the same, is suitable for use as an electrical insulating oil for general uses and is especially suitable for use in impregnation of oil-filled electrical appliances such as capacitors, oil-filled power cables and transformers.

As described at the beginning of this specification, the requirements of high-voltage withstanding and size reduction of such oil-filled electrical appliances have become severe in recent years. In order to meet these requirements, plastics are used to replace either partially or totally the conventional insulating paper as insulating materials or dielectric materials for the oil-filled electrical appliances. More particularly, as electrical insulating materials (dielectric materials) of electric capacitors, there is proposed the use of a combination of insulating paper and plastics films. As the plastics films, stretched or nonstretched polypropylene, polymethylpentene, polyester, polyvinylidene fluoride and polycarbonate films can be used. The use of these plastics films singly, the use of the embossed films of these plastics films to facilitate impregnation with the insulating oil, or the use of single-side or double-side metallized plastics films, where the metallic layer serves as an electrode, is also possible. In the case of oil-filled cables, the electrical insulating materials are made of polyolefin films such as cross-linked or non-cross-linked polyethylene film, stretched or nonstretched polypropylene film, and polymethylpentene film; paper-polyolefin laminated film made by the extrusion of polyolefin onto paper; composite film that is made by cross-linking insulating paper with silane-grafted polyethylene in the presence of a silanol condensation catalyst; or an artificial paper sheet that is made by mixing wood pulp and polyolefin fiber.

When an electric capacitor is provided with an insulating (dielectric) material that is partially or totally made of plastics, especially polyolefin, and when it is impregnated with the electrical insulating oil (including the electrical insulating substance itself) prepared according to the present invention, the insulating material can be fully and completely impregnated with the electrical insulating oil. This is because swelling of the insulating material is slight, and voids (unimpregnated portions) are not formed. Accordingly, corona discharge due to the convergence of electric fields to the voids hardly occurs, and dielectric breakdown can be well avoided. Furthermore, the electrical insulating oil prepared according to this invention has excellent hydrogen gas absorbing capacity and corona discharge resistance under high-voltage stress, so that it is possible to obtain both a long service life and high-voltage use of the electrical appliances.

In the case of electric power cables, a change in dimensions of the insulating material due to swelling is small, and resistance to the insulating oil flow can be made low so that oil impregnation can be performed in a short time. Of course, it will be understood that, because of the ease of impregnation, voids are hardly formed and the dielectric breakdown voltage becomes higher. When a cable is made by using an insulating material of a laminated film or composite film made of plastics material and paper, peeling, creasing and buckling of the insulating material upon bending of the cable do not occur even when the insulating material has been in contact with the electrical insulating oil for a long time. Further, as in the case of the electric capacitor, a power cable having a good corona discharge resistance can be obtained due to the excellent hydrogen gas absorbing capacity of the electrical insulating oil. Accordingly, it is also possible to obtain a long service life and high-voltage use, as for the capacitors.

According to this invention, the above-described advantageous features can be improved by impregnation with the electrical insulating oil consisting of a plurality of specific component materials,

5

owing to the synergistic effect between the component materials. Further, the good electrical characteristics, biodegradability, thermal resistance, and oxidation stability of each component material can be well maintained, and at the same time, the viscosity and pour point of the electrical insulating oil composition can be adjusted within desired ranges. Therefore, the manufacture of oil-filled electrical appliances is facilitated, and oil-filled electrical appliances exhibiting high performance under any use conditions can be obtained.

In the following, the electrical insulating oil and electrical appliances impregnated therewith according to the process of this invention will be described in more detail with reference to several examples.

Example 1

Monoisopropylbiphenyl was dehydrogenated in the presence of a catalyst and steam under the following conditions and obtained an electrical insulating substance containing 51 wt.% of monoisopropenylbiphenyl.

Conditions of dehydrogenation

Catalyst: Iron oxide catalyst containing promoters of potassium carbonate and chromium oxide
Trade mark: G64A, made by Nissan Girdler Catalyst Co., Ltd.
Particle size: 14—28 mesh (1.168 to 0.589 mm).
Temperature: 590°C.
LHSV: 1.0.
$H_2O$/starting material (by weight): 3.0.
Pressure: Atmospheric pressure.

In the next step, the dehydrogenation product was diluted by the starting material of monoisopropylbiphenyl, thereby preparing an electrical insulating oil containing 10 wt.% of monoisopropenylbiphenyl.

Capacitors were impregnated with this electrical insulating oil and the starting material of monoisopropylbiphenyl, respectively. The performances of both kinds of the capacitors were compared.

The capacitors were made by winding two-ply capacitor-use polypropylene film (each 14 μm thickness) as dielectric material and aluminum foil as electrode. The capacitance of the obtained capacitor after impregnation was about 0.4 μF.

Example 2

Dehydrogenation was carried out by using 1 - phenyl - 1 - xylylethane in like manner as Example 1 except that the reaction temperature was 420°C, to obtain a reaction mixture of electrical insulating substance containing 10 wt.% of 1 - phenyl - 1 - xylylethylene. Capacitors that were made in like manner to that of Example 1 were impregnated with the above-obtained electrical insulating substance and the 1 - phenyl - 1 - xylylethane, respectively, and the performances of both the capacitors were compared.

Example 3

Dehydrogenation was carried out by using diisopropylnaphthalene in like manner to that of Example 1 except that the reaction temperature was 500°C, to obtain a dehydrogenation product of electrical insulating substance having a bromine number of 8.1 cg/g. Assuming that all olefins are monoolefins, this value corresponds to 10.6 wt.% of olefins.

Capacitors were made through the procedure described below, and they were impregnated with the above electrical insulating substance and the starting material of diisopropylnaphthalene, respectively. The performances of both the capacitors were compared.

In the preparation of capacitors, capacitor-use polypropylene film (28 μm in thickness, 62 mm in width) and insulating paper (12 μm in thickness, 62 mm in width) were wound to form two-ply dielectric material. As electrodes, aluminum foil (7 μm in thickness, 50 mm in width) was wound. The capacitance of the obtained capacitors after impregnation was 0.7 μF.

In the performance tests, corona starting voltages (CSV), corona ending voltages (CEV) and breakdown times under a fixed voltage were measured. The results of the tests are shown in Table 1. In the tests for breakdown times, each value was calculated such that seven capacitors impregnated with the same oil were tested, and the maximum value and minimum value were neglected, and the average of the other five breakdown times was adopted as the resultant value. Furthermore, the breakdown times were represented by the values relative to the values of the starting materials for dehydrogenation.

TABLE 1

| Example No. | Impregnating oil | Kinematic viscosity (mm²/S (cSt) at 30°C) | CSV (kV) | CEV (kV) | Breakdown time (relative value) |
|---|---|---|---|---|---|
| 1 | Monoisopropylbiphenyl | 6.15 | 2.6 | 2.1 | 1.0 |
| | Monoisopropylbiphenyl plus dehydrogenation product (10 wt.% monoisopropenylbiphenyl) | 6.18 | 3.1 | 2.5 | 18.0 |
| 2 | 1-phenyl-1-xylylethane | 6.54 | 2.8 | 2.2 | 1.0 |
| | Dehydrogenation product (10 wt.% 1-phenyl-1-xylylethylene) | 6.55 | 3.2 | 2.6 | 18.5 |
| 3 | Diisopropylnaphthalene | 9.72 | 1.9 | 1.1 | 1.0 |
| | Dehydrogenation product | 9.79 | 2.9 | 2.1 | 25.4 |

Example 4

Dehydrogenation was carried out by using 1,1 - diphenylethane as in Example 1 except that the reaction temperature was 575°C, to obtain a reaction mixture containing 83 wt.% of diphenylethylene.

By using an electrical insulating oil containing 10 parts by weight of this reaction mixture and 90 parts by weight of 1 - phenyl - 1 - xylylethane, capacitors were made in like manner to that of Example 1. The performances of the capacitors were tested and the results of the tests are shown in Table 2.

Example 5

By using 1 - phenyl - 1 - (4' - ethylphenyl)ethane, dehydrogenation was carried out as in Example 1 except that the reaction temperature was 550°C, to obtain a reaction product having the following composition.

| Compounds | wt.% |
|---|---|
| 1-phenyl-1-(4'-ethylphenyl)ethane | 23.8 |
| 1-phenyl-1-(4'-ethylphenyl)ethylene | 39.9 |
| 1-phenyl-1-(4'-vinylphenyl)ethane | 5.0 |
| 1-phenyl-1-(4'-vinylphenyl)ethylene | 28.1 |
| Others | 3.2 |
| Total | 100.0 |

By using an electrical insulating oil containing 10 parts by weight of this reaction product and 90 parts by weight of 1 - phenyl - 1 - xylylethane, capacitors were made as in Example 1. The performances of the capacitors were tested and the test results are shown in Table 2. Incidentally, the values on breakdown times in Table 2 are all relative values with respect to the value in which the basic oil, 1 - phenyl - 1 - xylylethane, is singly used.

It will be understood from the results shown in Table 1 and 2 that the electrical insulating substance prepared according to this invention obtained by dehydrogenation can be used as it stands or in a mixture with other electrical insulating oils, and it exhibits quite excellent electrical properties.

In other words, the aromatic hydrocarbons used as the starting materials of dehydrogenation in the foregoing examples were used as electrical insulating oils and were regarded as suitable for use in the impregnation of oil-filled capacitors and power cables containing at least partly plastic materials. It will be apparent, however, that the electrical insulating oils using the electrical insulating substance prepared according to the present invention are superior to these oils.

TABLE 2

| Example No. | Impregnating oil | | | | Kinematic viscosity (cSt) (mm²/S) (at 30°C) | CSV (kV) | CEV (kV) | Breakdown time (relative value) |
|---|---|---|---|---|---|---|---|---|
| | Aromatic hydrocarbon | wt.% | Aromatic olefin | wt.% | | | | |
| 4 | 1-Phenyl-1-xylylethane | 90 | Dehydrogenation product of Ex. 4 | 10 | 6.12 | 3.2 | 2.6 | 19.3 |
| 5 | 1-Phenyl-1-xylylethane | 90 | Dehydrogenation product of Ex. 5 | 10 | 6.33 | 3.3 | 2.7 | 25.6 |

Example 6

In a process for preparing ethylbenzene, which is used for production of polystyrene, benzene was reacted with ethylene in the presence of an aluminum chloride catalyst. From the reaction mixture in this process, unreacted benzene, ethylbenzene and polyethylbenzene were distilled off by reduced pressure distillation to obtain a fraction having a boiling range of 260 to 310°C (normal pressure).

The main components in this fraction were 37 wt.% of 1,1 - diphenylethane and 32 wt.% of 1 - phenyl - 1 - ethylphenylethane. Besides them, 1,1 - di(ethylphenyl)ethane, Tetralin®, indane, naphthalene, fluorene, their alkyl derivatives, and unknown substances were also contained in the fraction.

The recovered fraction was dehydrogenated in the presence of a catalyst and steam under the following conditions.

Catalyst: Iron oxide catalyst containing promotors of potassium carbonate and chromium oxide.
Trade mark: G64A, made by Nissan Girdler Catalyst Co., Ltd.

Temperature: 500°C.

LHSV: 1.0.

$H_2O$/starting material: 3.0 (molar ratio).

Pressure: Atmospheric pressure.

After the dehydrogenation, lighter components and heavier components were removed to obtain a dehydrogenation product. The bromine number of this dehydrogenation product was 15.3 cg/g. Assuming that contained olefins are all monoolefins, this value corresponds to about 19 wt.% in olefin content. This dehydrogenation product was used as an electrical insulating oil for the following capacitor tests.

The dehydrogenated product and the foregoing recovered fraction before dehydrogenation were used as electrical insualting oils and capacitors were impregnated with them as in Example 1. The performances of the capacitors were evaluated by corona starting voltages (CSV), corona ending voltages (CEV) and breakdown times.

The capacitors were made by winding two-ply capacitor-use polypropylene film (each 14 μm thickness) as dielectric material and aluminum foil as electrodes. The capacitance of the obtained capacitors after impregnation was 0.4 μF. The breakdown times were represented by values relative to those of the recovered fraction before dehydrogenation. In the capacitor tests, 0.2 wt.% of BHT as an antioxidant was added to both the oils. The results are shown in the following Table 3.

TABLE 3

| Impregnation oil | CSV (kV) | CEV (kV) | Breakdown time (relative value) |
|---|---|---|---|
| Recovered fraction before dehydrogenation | 2.6 | 2.0 | 1 |
| Dehydrogenation product | 3.1 | 2.5 | 16.3 |

Electrical characteristics test

With regard to the electrical insulating oils prepared according to this invention that were used for the impregnation in the foregoing Examples 1 to 6, respectively, some electrical characteristics as insulating oils were measured. The results are shown in Table 4.

## EP 0 115 065 B1

TABLE 4

| | Electrical characteristics | | |
|---|---|---|---|
| Example No. | $\varepsilon$ at 80°C | tan δ %, at 80°C | $\rho$ $\Omega \cdot$ cm at 80°C |
| 1 | 2.52 | 0.019 | $9.1 \times 10^{14}$ |
| 2 | 2.51 | 0.017 | $8.9 \times 10^{14}$ |
| 3 | 2.56 | 0.021 | $9.4 \times 10^{14}$ |
| 4 | 2.51 | 0.019 | $8.8 \times 10^{14}$ |
| 5 | 2.50 | 0.022 | $9.3 \times 10^{14}$ |
| 6 | 2.49 | 0.023 | $9.0 \times 10^{14}$ |

### Claims

1. A process for preparing an electrical appliance which contains or is impregnated with an electrical insulating oil, wherein the apparatus is filled or impregnated with a dehydrogenated reaction mixture containing more than 0.5% by weight of an aromatic monoolefin or diolefin having two condensed or noncondensed aromatic nuclei obtained by dehydrogenating an aromatic hydrocarbon or hydrocarbons each having two condensed or noncondensed aromatic nuclei and an aliphatic or alicyclic hydrocarbon residual group or groups having two or more carbon atoms at a temperature in the range of 350 to 650°C in the presence of a dehydrogenation catalyst, without further adding a diarylalkane.

2. The process according to claim 1, wherein said electrical appliance is one member selected from the group consisting of an oil-filled capacitor, an oil-filled cable and a transformer.

3. The process according to claim 1, wherein the electrical insulating material or dielectric material used in said electrical appliance is cellulose film or plastics film or their combination.

4. The process according to claim 3, wherein said plastics film is made of polyethylene or polypropylene.

### Patentansprüche

1. Verfahren zur Herstellung einer elektrischen Vorrichtung die ein elektrisch isolierendes Öl enthält oder mit einem solchen getränkt ist, wobei die Vorrichtung mit einem mehr als 0,5 Gew.-% eines aromatischen Monoolefins oder Diolefins mit zwei kondensierten oder nichtkondensierten aromatischen Kernen enthaltenden dehydrierten Reaktionsgemisch gefüllt oder getränkt ist, das durch Dehydrieren einer oder mehrerer aromatischer Kohlenwasserstoffe, die jeweils zwei kondensierte oder nichtkondensierte aromatische Kerne und eine oder mehrere aliphatische oder alicyclische Kohlenwasserstoffrestgruppen mit zwei oder mehreren Kohlenstoffatomen enthalten, bei einer Temperatur innerhalb des Bereiches von 350 bis 650°C in Gegenwart eines Dehydrierungskatalysators ohne weiteren Zusatz eines Diarylalkans erhalten wird.

2. Verfahren nach Anspruch 1, wobei die elektrische Vorrichtung ein Teil ist, das aus der einen ölgefüllten Kondensator, ein ölgefülltes Kabel und einen Transformator umfassenden Gruppe ausgewählt ist.

3. Verfahren nach Anspruch 1, wobei das in dieser elektrischen Vorrichtung verwendete elektrische isolierende Material oder dielektrische Material ein Cellulosefilm oder ein Kunststofffilm oder eine Kombination aus diesen ist.

4. Verfahren nach Anspruch 3, wobei der Kunststofffilm aus Polyethylen oder Polypropylen hergestellt ist.

### Revendications

1. Procédé de fabrication d'un appareil électrique qui contient ou est imprégné avec une huile isolante électrique, dans lequel l'appareil est rempli ou imprégné par un mélange réactionnel de déshydrogénation contenant plus de 0,5% en poids d'une monooléfine ou dioléfine contenant deux noyaux aromatiques condensés ou non, obtenu par déshydrogénation d'un ou de plusieurs hydrocarbures aromatiques possédant chacun deux noyaux aromatiques condensés ou non, et un ou plusieurs groupes résiduels hydrocarbonés aliphatiques ou alicycliques possédant deux ou plusieurs atomes de carbone, à une température comprise entre 350 et 650°C en présence d'un catalyseur de déshydrogénation, sans addition ultérieure d'un diarylalcane.

2. Procédé conforme à la revendication 1, dans lequel ledit appareil électrique est un élément choisi dans le groupe constitué par les condensateurs remplis d'huile, les câbles remplis d'huile, et les transformateurs.

3. Procédé conforme à la revendication 1, dans lequel le matériau isolant électrique ou matériau diélectrique, utilisé dans ledit appareil électrique est un film de cellulose ou un film de matière plastique, ou leur combinaison.

4. Procédé conforme à la revendication 3, dans lequel ledit film de matière plastique est réalisé en polyéthylène ou en polypropylène.